# EUROPÄISCHE PATENTSCHRIFT

(11) **EP 2 833 816 B1**
(45) Veröffentlichungstag und Bekanntmachung des Hinweises auf die Patenterteilung: **27.02.2019**
(21) Anmeldenummer: 13717227.6
(22) Anmeldetag: 05.04.2013
(51) Int. Cl.: A61B 18/12, A61B 5/00, A61G 3/00, A61B 5/0402, A61G 12/00, A61M 1/00, A61N 1/00, A61B 8/00, A61N 1/39

(54) **MODULARES MEDIZINISCHES GERÄT**
MODULAR MEDICAL APPLIANCE
APPAREIL MÉDICAL MODULAIRE

(30) Priorität: 05.04.2012 DE 102012103029
(43) Veröffentlichungstag der Anmeldung: 11.02.2015
(73) Patentinhaber: Kagan, Eugen, 53859 Niederkassel Lülsdorf (DE)
(72) Erfinder: Kagan, Eugen, 53859 Niederkassel Lülsdorf (DE)
(74) Vertreter: Wagner Albiger & Partner Patentanwälte mbB
(86) Internationale Anmeldenummer: PCT/EP2013/057188
(87) Internationale Veröffentlichungsnummer: WO 2013/150135

(56) Entgegenhaltungen:
- WO-A1-2008/140528
- DE-C1- 19 922 855
- US-A- 4 715 385
- US-A- 5 590 648
- US-A- 5 701 894
- US-A1- 2004 249 279

## Beschreibung

Die Erfindung betrifft ein modulares medizinisches Gerät, beispielsweise einsetzbar als modulares Intensivtherapiegerät, umfassend ein Basismodul mit einer Vorderseite und einer Rückseite, sowie mehrere mit dem Basismodul in Wirkverbindung bringbare Funktionsmodule, die eine intensivmedizinische Therapiefunktionalität zur aktiven Therapie akuter Störungen der Vitalfunktionen aufweisen.

Derartige modulare Intensivtherapiegeräte sind bekannt, wozu beispielsweise auf die US 5590648 verwiesen wird.

Sie dienen dazu, einen Patienten mit entsprechendem Therapiebedarf individuell versorgen zu können, indem ein Basismodul mit den vom Patienten benötigten Funktionsmodulen, wie einem Beatmungsmodul für die Beatmung oder einem Defibrillator, um die individuell erforderliche Behandlung und Therapie der Vitalfunktionen eines Patienten sicher zu stellen. Der Vorteil eines solchen modularen Intensivtherapiegerätes liegt von daher darin, dass die für den jeweiligen Patienten benötigte Ausrüstung platzsparend in einer Einheit zusammen gestellt, bei sich änderndem Therapiebedarf ergänzt oder verringert werden kann und im Falle eines Defektes eines einzelnen Funktionsmoduls ein leichter und schneller Austausch desselben möglich ist.

Die bekannten modularen Intensivtherapiegeräte der eingangs genannten Art weisen jedoch den Nachteil auf, dass sie sich aufgrund ihrer großen Abmessungen vornehmlich für den stationären Einsatz eignen, so dass eine Verwendung für die Notfallversorgung, z.B. als Ausrüstungsgegenstand für ein Notarztfahrzeug nicht möglich ist.

Aus der EP 0673223 B1 sowie der Firmenschrift "Sino-Hero (Shenzen) Bio-Medical Electronics Co., Ltd., China: AcuitSign M8 - Modular Patient Monitor; 24.01.2011" sind tragbare, modular aufgebaute Patientenüberwachungseinheiten bekannt, die zwar kompakt und mit einer Notstromversorgung für kurzzeitigen Betrieb, z.B. den Transport eines Patienten innerhalb eines Krankenhauses ausgerüstet sind. Diese bekannten Vorrichtungen weisen jedoch keine für die Therapie des Patienten bereitstellbaren Funktionsmodule auf und eignen sich nicht für die an den Rettungs- bzw. Notfalleinsatz anschließende stationäre Behandlung des Patienten. Dies bringt den Nachteil mit sich, dass der Patient nach Eintreffen in einem Krankenhaus an die dort stationär vorgehaltenen Intensivtherapiegeräte angeschlossen werden muss, d.h. sämtliche bereits im Rahmen der Notfallversorgung gelegten Zugänge und Sonden zum Patienten müssen neu verkabelt und angeschlossen werden. Dies erscheint verbesserungswürdig.

Ebenfalls zeigt die US 2012/0029304 und die DE19922855 eine modular aufgebaute Patientenüberwachungseinheit.

Derartige modular aufgebaute Überwachungsgeräte sind in der klinischen Praxis nicht üblich, sondern es ist eine Mehrzahl von einzelnen Geräten, wie z. B. Beatmungsgerät, Defibrillator, Patientenüberwachungsgerät, mehrstufige Infusionsgeräte etc., für Therapie und Überwachung des Patienten notwendig, woraus sich eine Vielzahl von Nachteilen ergeben:
a) Diese platzieren sich in der Intensivstation von beiden Seiten des Patientenbettes und erschweren den freien Zutritt zum Patienten.
b) Diese benötigen separate Kontrolle und Steuerung, von allen Seiten des Patientenbettes.
c) Diese kommunizieren nicht mit einander und können nicht automatisch die interdependenten Einstellungen verändern. Die gemessenen Parameter haben keine Auswirkung auf die Therapie. Z. B. misst das Patientenüberwachungsgerät den CO2-Gehalt des Patienten und der Arzt verändert die Sauerstoffzufuhr an dem Beatmungsgerät.
d) Nur Patientenüberwachungsgeräte können sich mit der Überwachungszentralstation der Krankenschwesternzimmer (z. B. in der Intensivtherapieabteilung) verbinden um Daten (z. B. Vitalparameter) zu übertragen oder Alarm auszulösen. Alle weiteren Geräte (z. B. Beatmungsgerät, Defibrillator, Infusionsgerät etc.) besitzen diese Möglichkeit nicht. Wenn darüber hinaus die Patientenüberwachungsgeräte von unterschiedlichen Herstellern stammen, kommunizieren diese nur bedingt mit der Überwachungszentralstation des Krankenhauses, da unterschiedliche Hersteller unterschiedliche Software-Protokolle haben.
e) Diese sind nicht im Stande, jederzeit die Patientendatenfernübertragung innerhalb des Krankenhauses (z. B. Innenkrankenhaustransport) oder außerhalb des Krankenhauses (z. B. Außenkrankenhaustransport) für die Befunderstellung oder Überwachung bereitzustellen.
f) Diese kommunizieren nur bedingt mit dem Archivierungsnetz des Krankenhauses (z. B. Kommunikationssystem PACS), da unterschiedliche Hersteller unterschiedliche Software-Protokolle nutzen und die Verbindung inkompatibel sein kann.
g) Diese sind entweder Stationär vorgesehen oder als Transportgerät ausgeführt. Die stationären Geräte sind für den Transport nicht geeignet, die Transportgeräte sind sehr limitiert und können nicht für die Langzeittherapie eingesetzt werden.
h) Diese sind beim Notfalleinsatz im Feld, beim Transport (Krankenwagen, Helikopter, Flugzeug etc.) sowie in der Aufnahmestation und Intensivstation (OP und Aufwachraum) mehrmals in jedem Krankenhaus vorhanden, wodurch der Patient durchgehend an die gleichartigen Geräte mehrmals neuangeschlossen wird. Das Krankenhaus muss jede Station mit den gleichartigen Geräten versorgen.

Aufgabe der Erfindung ist es, ein modulares Intensivtherapiegerät der eingangs genannten Art vorzuschlagen, weiches größtmögliche Flexibilität hinsichtlich der am Basismodul befestigbaren Funktionsmodule gewährleistet und gleichermaßen für den mobilen Einsatz im Rahmen der Notfallversorgung wie auch für den stationären Einsatz im Rahmen der Intensivtherapiebehandlung innerhalb eines Krankenhauses oder dergleichen geeignet ist.

Zur Lösung der gestellten Aufgabe wird erfindungsgemäß die Ausgestaltung eines modularen Intensivtherapiegerätes mit den Merkmalen des Patentanspruches 1 vorgeschlagen.

Vorteilhafte Ausgestaltungen und Einzelheiten der Erfindung sind Gegenstand der abhängigen Ansprüche.

Die Erfindung schlägt vor, das Basismodul im Bereich der Vorderseite desselben mit einer Anzeige- und Bedieneinheit und im Bereich der Rückseite desselben mit Aufnahmevorrichtungen für die Funktionsmodule auszustatten, mittels derer die Funktionsmodule an dem Basismodul lösbar befestigbar und mit diesem elektrisch verbindbar sind. Das Intensivtherapiegerät kann auf diese Weise als kompakte von Hand tragbare Einheit ausgebildet werden, die beispielsweise von einer Person im Rahmen eines Notfalleinsatzes zum Patienten gebracht, dort in Betrieb genommen und während des Transports des Patienten zum Krankenhaus fortlaufend in Betrieb gehalten wird und den Patienten bedarfsgerecht therapiert, z.B. beatmet und/oder als Defibrillator bzw. Schrittmacher auf dessen Herzfunktion einwirkt. Nach Ankunft des Patienten im Krankenhaus kann die Intensivtherapiebehandlung ununterbrochen mit dem erfindungsgemäßen modularen Intensivtherapiegerät fortgesetzt werden, da sämtliche für die Behandlung des Patienten notwendigen Funktionsmodule mit dem Basismodul in Wirkverbindung stehen und je nach Therapiebedarf des Patienten auch weitere Funktionsmodule hinzugefügt bzw. nicht mehr benötigte Funktionsmodule entfernt werden können. Die unterschiedlichen nach dem Prinzip des Plug & Play arbeitenden Module können auch zwischen den einzelnen Basisgeräten innerhalb des ganzen Krankenhauses nach Bedarf ausgetauscht werden. Es besteht von daher trotz der Kompaktheit des erfindungsgemäßen modularen Intensivtherapiegerätes keine Notwendigkeit mehr, den Patienten nach seiner Ankunft im Krankenhaus neu zu verkabeln oder mit dort vorgehaltenen Intensivtherapiegeräten zu verbinden.

Das vorgeschlagene modulare Intensivtherapiegerät kann unterschiedliche Funktionsmodule aufweisen, die z. B. folgende Geräte einbeziehen:
Beatmungsgerät → Beatmungsmodul
Defibrillator → Defibrillatormodul
Infusionsgerät → Infusionsmodul
Elektroenzephalograf → Elektroenzephalografmodul
Ultraschalldiagnostikgerät → Ultraschalldiagnostikmodul
Hochfrequenzchirurgiegerät → Hochfrequenzchirurgiemodul
sowie weitere beliebige in der Intensivtherapie und Chirurgie einsetzbare Geräte.

Insoweit ist es möglich, dass das im Rahmen der Notfallversorgung zum Einsatz kommende erfindungsgemäße Intensivtherapiegerät während des gesamten Krankenhausaufenthaltes des Patienten einschließlich der intensivmedizinischen Versorgung bei diesem verbleibt. Darüber hinaus wird jederzeit die Patientendatenfernübertragung mittels WLAN, GPS, GMS, Bluetooth, Infrarot etc. innerhalb des Krankenhauses z. B. auch beim Innenkrankenhaustransport oder außerhalb des Krankenhauses z. B. auch beim Außenkrankenhaustransport für die Befunderstellung oder Überwachung bereitgestellt.

Ein weiteres Modul kann als Patientenüberwachungsmodul ausgeführt werden und die Funktionen eines Patientenüberwachungsgerätes in sich vereinen.

Mit Vorteil sind die Funktionsmodule jeweils in separaten Gehäusen aufgenommen, deren Abmessungen einer Vereinheitlichung in Bezug auf den an der Rückseite des Basismoduls vorgehaltenen Platz für die Verbindung mit dem Basismodul unterliegen. Beispielsweise können alle Gehäuse der Funktionsmodule in gleicher Größe ausgebildet sein, oder aber die Gehäuseabmessungen unterliegen einem vorgegebenen Rastermaß, wobei je nach Platzbedarf die Größe jedes Moduls entsprechend ausgeführt werden kann, so dass sie universell und in frei wählbarer Konfiguration mit dem Basismodul verbindbar sind.

Die Funktionsmodule sind nach einer bevorzugten Ausführungsform der Erfindung für sich alleine nicht autark arbeitsfähig, sondern erlangen ihre Funktionsfähigkeit erst mit Anbringung an und elektrischer Verbindung mit dem Basismodul.

Nach einem Vorschlag der Erfindung weist das Basismodul des modularen Intensivtherapiegerätes eine über die Rückseite überstehende Basiseinheit auf, die eine Stromversorgungseinheit und eine Datenverarbeitungseinheit umfasst. Die Stromversorgungseinheit stellt die Stromversorgung sowohl für das Basismodul als auch für die elektrisch mit diesem verbundenen Funktionsmodule sicher, wobei im Rahmen der universellen Verwendbarkeit des erfindungsgemäßen modularen Intensivtherapiegerätes sowohl im Notfalleinsatz als auch stationär im Krankenhaus die Stromversorgungseinheit sowohl über einen handelsüblichen Netzanschluss, insbesondere 220 V Wechselstrom, wie auch über einen in Rettungsfahrzeugen üblichen Stromanschluss, beispielsweise 12 oder 24 V Gleichstrom verfügt.

Darüber hinaus ist vorgesehen, einen korrespondierend zu den Funktionsmodulen ausgebildeten und alternativ oder zusätzlich zu den Funktionsmodulen an einer Aufnahmevorrichtung des Basismoduls befestigbaren und mit diesem elektrisch verbindbaren Akkumulator für die mobile Stromversorgung des Intensivtherapiegerätes vorzusehen, der während der Notfallversorgung und während des Transports des Patienten zum Einsatz kommen kann.

Die Datenverarbeitungseinheit dient als zentrale Steuer- und Regelungseinheit für das gesamte modulare Intensivtherapiegerät, d.h. sie kommuniziert einerseits mit der im Basismodul vorgehaltenen Anzeige und Bedieneinheit, andererseits aber auch mit sämtlichen am Basismodul befestigbaren und konnektierbaren Funktionsmodulen und korrespondierenden Modulen, etwa dem Akkumulator und dem Überwachungsmodul, die sich beim Anschließen an das Basismodul an der Datenverarbeitungseinrichtung entsprechend anmelden und von der Datenverarbeitungseinheit sodann gesteuert werden. Die Datenverarbeitungseinheit kann darüber hinaus diverse Schnittstellen für den Anschluss externer Geräte umfassen, beispielsweise USB-Schnittstellen, RS 232- und Ethernet-Schnittstellen, wie auch Lesegeräte für Speicherkarten und dergleichen mehr. Auch können Eingänge beispielsweise für ein Schwesternrufsystem oder zentrales Krankenhauskommunikationsnetz vorgehalten werden. Darüber hinaus wird jederzeit die Patientendatenfernübertragung mittels WLAN, GPS, GMS, Bluetooth, Infrarot etc. bereitgestellt.

Die Basiseinheit weist laut Erfindung vorzugsweise eine über die Rückseite des Basismoduls vorstehende Länge auf, die der Breite der Funktionsmodule entspricht, so dass sich das Basismodul mit seiner rückseitig vorstehenden Basiseinheit und den am Basismodul befestigten Funktionsmodulen idealerweise zu einem quaderförmigen Gehäusekörper ergänzen, der robust, platzsparend und leicht von Hand zu tragen ist.

Weiterhin ist bevorzugt, dass die Basiseinheit die Unterseite des modularen Intensivtherapiegerätes bildet, so dass sie in Bezug auf das Basismodul eine verbreiterte Standfläche für das modulare Intensivtherapiegerät bildet.

Die Aufnahmevorrichtungen für die Funktionsmodule können beispielsweise eine Vielzahl von parallel zueinander im Bereich der Rückseite des Basismoduls verlaufende Führungsschienen für komplementär ausgebildete Einsteckvorsprünge der Funktionsmodule sein, so dass die Funktionsmodule mit den Einsteckvorsprüngen leicht in die Führungsschienen einschiebbar sind. Die elektrischen Verbindungsschnittstellen für die Funktionsmodule sind in einem solchen Falle in einem Anschlussbalken angeordnet, der einen Teil des Basismoduls bildet und zugleich einen Endanschlag für die in die Führungsschienen einschiebbaren Funktionsmodule bildet, so dass die Funktionsmodule beim Erreichen des Endanschlages aufgrund des Einschiebens in die Führungsschienen selbsttätig und zuverlässig mit dem Basismodul elektrisch verbunden werden. Die Funktionsmodule des modularen Intensivtherapiegerätes gemäß der Erfindung unterliegen keiner generellen Beschränkung und können vom Fachmann individuell je nach Therapieausrichtung ausgewählt werden. Für den Einsatz als modulares Intensivgerät beispielsweise für die Ausrüstung eines Rettungsfahrzeuges wird es für sinnvoll erachtet, Funktionsmodule in Form eines Defibrillatormoduls und eines Beatmungsmoduls vorzusehen.

Ferner kann ein korrespondierend zu den Funktionsmodulen (11) ausgebildetes und alternativ oder zusätzlich zu den Funktionsmodulen (11) an einer Aufnahmevorrichtung des Basismoduls (10) befestigbares und mit diesem elektrisch verbindbares Patientenüberwachungsmodul zur Überwachung von Vitalfunktionen des Patienten vorgesehen sein, um mittels des erfindungsgemäßen modularen Intensivtherapiegerätes auch eine ausreichende Überwachung der Vitalfunktionen des Patienten sowie eine davon abhängige Steuerung der Funktionsmodule zu gewährleisten.

Ein solches Patientenüberwachungsmodul für die Vitalfunktionen oder -parameter kann z.B. EKG-, Sp02-, Temperatur-, IBP- und/oder NIBP-Messungen ausführen, die gemessenen Parameter und Einstellungen überwachen, zugehörige einstellbare Grenzwerte überwachen und bei Über- bzw. Unterschreiten derselben Alarme erzeugen sowie die gemessenen Parameter darstellen und dokumentieren. Für die Dokumentation kommen z.B. Speichermedien und/oder Drucker in Frage.

Das Defibrillatormodul des erfindungsgemäßen Intensivtherapiegerätes kann manuelle, halb- und vollautomatische Betriebsweisen bieten und kann sowohl synchronisiert als auch unsynchronisiert betrieben werden. Weitere Modi umfassen das Monitoring gemessener Parameter und Einstellungen, unterschiedliche Impulsformen (Biphasisch, stromgesteuerter Impuls) sowie eine einstellbare Energie z.B. im Bereich von 0 bis 360 J und die Verwendung von Einweg-Klebeelektroden oder wiederverwendbaren Hard Paddles.

Das Beatmungsmodul des erfindungsgemäßen Intensivtherapiegerätes kann Modi für die Notfall- oder Langzeitbeatmung darstellen, jeweils abgestimmt auf Erwachsene, Kinder und Neugeborene. Es kann sowohl elektrisch als auch pneumatisch betrieben werden und das Monitoring von gemessenen Parametern und Einstellungen ermöglichen. Es kann sowohl für die invasive als auch die nichtinvasive Beatmung verwendet werden, wobei Beatmungsmodi, wie Volumen, Fluss und druckunterstützte Spontanbeatmung eingestellt werden können.

Ferner weist das Beatmungsmodul eine Steuerung mit einer Schnittstelle auf, an die ein Narkosegerät anschließbar und mittels der Steuerung des Beatmungsmoduls steuerbar ist. Die Funktionen des Beatmungsmoduls können damit auch in Verbindung mit einem ohne eigene Steuerung vorliegenden baulich vereinfachten Narkosegerät z.B. im Rahmen von Operationen eingesetzt werden in dem die Steuerung des Beatmungsmoduls mit dem Narkosegerät gekoppelt wird und auch dessen Steuerung übernimmt.

Durch die einfache Verkopplung des vorgeschlagenen modularen Intensivtherapiegeräts (mit Beatmungs- und Patientenüberwachungsmodul) mit einem dann baulich vereinfachten Narkosegerätewagen entsteht ein vollfunktionales und hochwertiges Narkosegerät. Dieser wird durch das Basisgerät angesteuert und durch das Beatmungsmodul angetrieben.

Das vorgeschlagene modulare Intensivtherapiegerät kann angeschlossen an den Patienten in den OP-Raum angeliefert, unkompliziert mit dem Narkosegerätewagen verknüpft/getrennt, sowie nach der Operation in den Aufwachraum weiter geführt werden. Dabei entfällt das mehrfache Anschließen des Patienten an mehrere-gleiche Geräte.

Das vorgeschlagene modulare Intensivtherapiegerät ermöglicht die Kommunikation zwischen den einzelnen Modulen desselben (Beatmungsgerät, Patientenüberwachungsgerät und Narkosegerät), wobei eine automatische Einstellung anhand der Auswertung von Parametern zustande kommt. Z. B. wird die Narkosegaszufuhr in Abhängigkeit von den ausgewerteten EKG-Daten automatisch eingestellt, so dass der Anästhesist nur die Einstellungen kontrollieren muss.

Durch den kompakten modularen Aufbau des Intensivtherapiegeräts (z. B. mit Beatmungsmodul, Patientenüberwachungsmodul und Narkosegerät) wird freier Platz in dem OP-Raum geschaffen, womit der Zugang des Personals zu dem Patienten erleichtert oder sogar ermöglicht wird.

Die Anzeige- und Bedieneinheit des erfindungsgemäßen modularen Intensivtherapiegerätes ist vorteilhaft von einem Touchscreen gebildet, so dass eine leichte und intuitive Bedienbarkeit auf einer grafischen Benutzeroberfläche ermöglicht wird. Es ist möglich, die Anzeige- und Bedieneinheit auch um einen oder mehrere Steuerknöpfe und dergleichen zu ergänzen.

Darüber hinaus kann zusätzlicher Funktionsumfang im modularen Intensivtherapiegerät eingebracht werden, beispielsweise ist es denkbar, Lautsprecher und Kameras im Basismodul oder einem Funktionsmodul anzuordnen, um eine visuelle Patientenüberwachung mit Interaktionsmöglichkeit zu schaffen.

Im Rahmen der Erfindung ist es somit möglich, ein kompaktes, flaches Wandgerät, das durch modularen Aufbau mehrere Geräte in sich vereint, welches sowohl für Transport sowie für stationären Bereich geeignet ist, auszubilden. Folgende Vorteile sind somit erzielbar:
a) Durch die Vereinigung von mehreren Geräten zu einem flachen Wandgerät wird der freie Zugang zum Patienten ermöglicht.
b) Das vorgeschlagene modulare Intensivtherapiegerät ermöglicht eine einheitliche Kontrolle und Steuerung, alle relevanten Parameter werden auf einem Bildschirm angezeigt, wodurch die Arbeit des Krankenhauspersonals erleichtert und verbessert wird.
c) Das vorgeschlagene modulare Intensivtherapiegerät ermöglicht die Kommunikation zwischen den einzelnen Modulen (Beatmungsmodul, Patientenüberwachungsmodul, Defibrillatormodul etc.), wobei eine automatische Einstellung anhand der Auswertung von Parametern zustande kommt. Die gemessenen Parameter haben eine direkte Auswirkung auf die Therapie. Z. B. misst das Patientenüberwachungsmodul den CO2-Gehalt des Patienten und das vorgeschlagene modulare Intensivtherapiegerät verändert die Sauerstoffzufuhr des Beatmungsmoduls automatisch. Der Arzt kann in diesem Fall die automatischen Veränderungen nur kontrollieren oder auch verändern.
d) Das vorgeschlagene modulare Intensivtherapiegerät kann die relevanten Daten und Alarmierungen von jedem einzelnen Modul (z. B. Beatmungsmodul, Patientenüberwachungsmodul, Defibrillatormodul, Infusionsmodul etc.) an die Überwachungszentralstation des Krankenschwesternzimmers (z. B. in der Intensivtherapieabteilung) übertragen. Somit kann die Krankenschwester an der Zentralstation alle lebenswichtigen Daten und Alarmierungen empfangen, nicht nur die von dem Patientenüberwachungsgerät. Mit der Patientendatenübertragung wird eine zweistufige Alarmierung an die Überwachungszentralstation des Krankenschwesternzimmers übertragen und an dem Überwachungsbildschirm das konkrete Gerät mit den betroffenen Parametern angezeigt. Darüber hinaus wird durch ein gemeinsames Software-Protokoll die Verbindung des modularen Intensivtherapiegeräts mit der Überwachungszentralstation des Krankenschwesternzimmers (z. B. in der Intensivtherapieabteilung) deutlich verbessert, wobei es unterschiedliche Patientendatenübertragungsmöglichkeiten wie z. B. mittels WLAN, LAN, GPS, GSM, Bluetooth, Infrarot etc. vorgesehen sind. Ferner kann mit Hilfe einer eingebauten Kamera das Echtzeitbild von dem Patienten an die Zentralstation übertragen werden.
e) Das vorgeschlagene modulare Intensivtherapiegerät ist im Stande, jederzeit die Patientendatenfernübertragung mittels WLAN, GPS, GMS, Bluetooth, Infrarot etc. innerhalb des Krankenhauses (z. B. Innenkrankenhaustransport) oder außerhalb des Krankenhauses (z. B. Außenkrankenhaustransport) für die Befunderstellung oder Patientenüberwachung (inklusive Echtzeitbild) bereitzustellen.
f) Durch ein gemeinsames Software-Protokoll wird die Verbindung des modularen Intensivtherapiegeräts mit dem Archivierungsnetz des Krankenhauses (z. B. Kommunikationssystem PACS) deutlich verbessert, wobei unterschiedliche Patientendatenübertragungsmöglichkeiten wie z. B. WLAN, LAN, GPS, GSM, Bluetooth, Infrarot etc. vorgesehen sind.
g) Das vorgeschlagene modulare Intensivtherapiegerät kann mit allen notwendigen Funktionen ausgestattet sein, die für Langzeittherapie und Überwachung des Patienten erforderlich sind. Das vorgeschlagene modulare Intensivtherapiegerät kann beim Transport (Außen- und Innenkrankenhaustransport) und im stationären Bereich eingesetzt werden.
h) Das vorgeschlagene modulare Intensivtherapiegerät kann beim Notfalleinsatz im Feld, beim Transport (Krankenwagen, Helikopter, Flugzeug etc.) sowie in der Aufnahmestation und Intensivstation (OP und Aufwachraum) patientenbegleitend eingesetzt werden, der Patient muss nur einmal angeschlossen werden.
i) In der Notaufnahme und der Intensivstation befinden sich mehrere Patientenbetten, wo alleinstehende Geräte (Beatmungsgerät, Patientenüberwachungsgerät, Defibrillator, Infusionsgeräte etc.) bei Bedarf eingesetzt werden. Durch den modularen Aufbau des vorgeschlagenen Intensivtherapiegerätes kann jeweils ein Basisgerät neben den Patientenbetten platziert werden und nur die unterschiedlichen Plug & Play Module zwischen den einzelnen Basisgeräten innerhalb des ganzen Krankenhauses nach Bedarf ausgetauscht werden, wodurch enorme Kostenvorteile für das Krankenhaus entstehen.

Die Erfindung wird nachfolgend anhand eines Ausführungsbeispiels in den Zeichnungen in weiteren Einzelheiten erläutert.

Es zeigen:
- Fig. 1: in perspektivischer Darstellung die Vorderansicht eines modularen Intensivtherapiegerätes gemäß der Erfindung.
- Fig. 2: in perspektivischer Darstellung die Rückansicht des modularen Intensivtherapiegerätes gemäß Figur 1.
- Fig. 3: in perspektivischer Darstellung die Seitenansicht des modularen Intensivtherapiegerätes gemäß Figur 1.
- Fig. 4: in perspektivischer Darstellung eine weitere Seitenansicht des modularen Intensivtherapiegerätes aus einer gegenüberliegenden Blickrichtung gegenüber der Darstellung in Figur 3.
- Fig. 5: die Einzelheit aus Figur 4 in vergrößerter Darstellung.
- Fig. 6: die Darstellung gem. Figur 2 mit nicht vollständig am Basismodul befestigten Funktionsmodulen.
- Fig. 7: das Basismodul gemäß der Darstellung in Figur 2 mit abgenommenen Funktionsmodulen.

Aus den Figuren ist ein mit Bezugsziffer 1 gekennzeichnetes modulares Intensivtherapiegerät ersichtlich, welches ein Basismodul 10 sowie eine Vielzahl von mit dem Basismodul 10 in nachfolgend noch beschriebener Weise in Wirkverbindung bringbare Funktionsmodule 11 oder korrespondierend zu den Funktionsmodulen ausgebildete weitere Module umfasst, die nachfolgend je nach ihrem Funktionsinhalt mit 11.1 bis 11.5 gekennzeichnet sind.

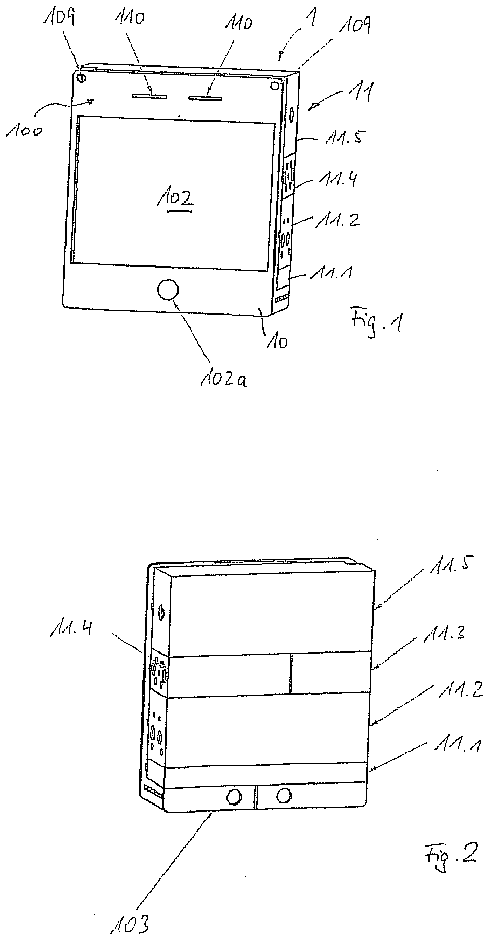

Im Bereich der aus der Figur 1 ersichtlichen Vorderseite 100 des Basismoduls 10 ist eine Anzeige- und Bedieneinheit 102 in Gestalt eines Touchscreens sowie ein Einstellknopf 102a vorgesehen, ergänzt durch Öffnungen 110 für Lautsprecher und 109 für Kameramodule.

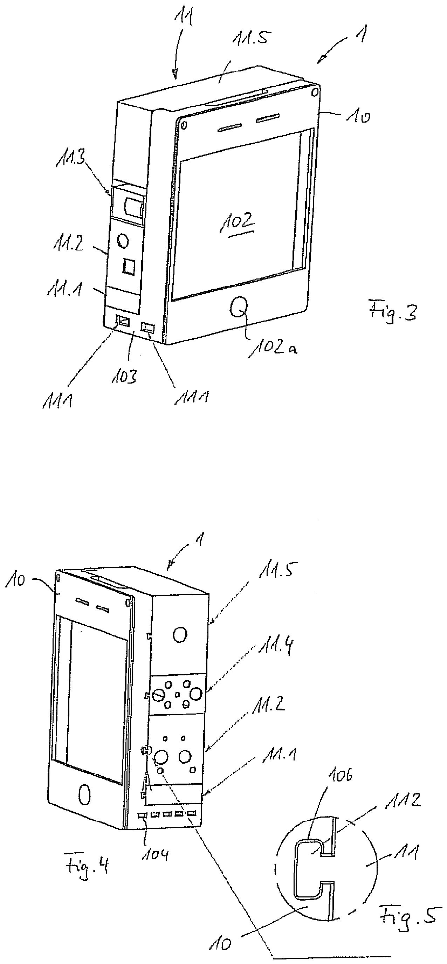

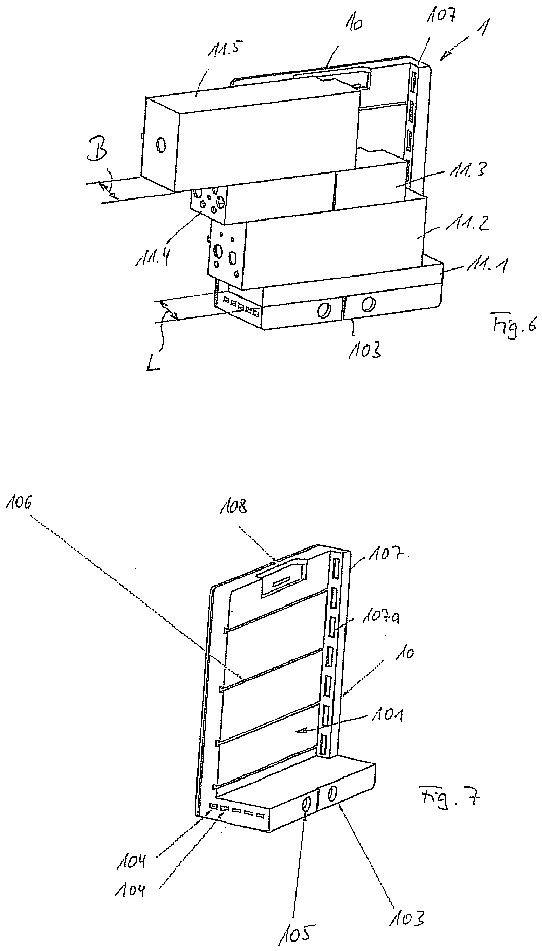

Wie insbesondere aus der Einzelteildarstellung gemäß Figur 7 ersichtlich, umfasst das Basismodul 10 darüber hinaus an seiner Oberseite einen Tragegriff 108, mit welchem es von Hand tragbar ist, ferner im Bereich der Rückseite 101 eine Vielzahl von parallel zueinander verlaufenden und in einem Rastermaß angeordnete Führungsschienen 106, die gemäß der vergrößerten Einzelteildarstellung in Figur 5 mit einem Hinterschnitt ausgebildet sind.

Die Unterseite des Basismoduls 10 bildet ein etwa L-förmig über die Rückseite 101 hervorstehende Basiseinheit 103, die sowohl eine Stromversorgungseinheit als auch eine Datenverarbeitungseinheit für das modulare Intensivtherapiegerät 1 beherbergt und insoweit integral mit dem Basismodui 10 verbunden ist.

Die Basiseinheit 103 bildet somit auch eine vergrößerte Standfläche für das Intensivtherapiegerät 1 und ist zur Kühlung der aufgenommenen Bauteile mit Lüfteröffnungen 105 sowie einer Vielzahl von Schnittstellen 104 für die Datenverarbeitungseinheit, beispielweise USB, HDMI und RS232 sowie Ethernet und auch mit Netzanschlussbuchsen 111 für die Stromversorgungseinheit, beispielsweise für 220 V Wechselstrom sowie 12 oder 24 V Gleichstrom versehen.

Um dem modularen Intensivtherapiegerät 1 mit seinem Basismodul 10 die für die Intensivtherapie eines Patienten erforderliche Funktionalität zu verleihen, wird das Basismodul 10 je nach Bedarf und insoweit individuell mit einem oder mehreren Funktionsmodulen 11 ausgerüstet, die jeweils in einem separaten Gehäuse aufgenommen sind und bei Verbindung mit dem Basismodul 10 durch die im Basismodul vorgehaltene Stromversorgungseinrichtung, Datenverarbeitungseinrichtung sowie Anzeige- und Bedieneinheit die gewünschte Funktion für die Patientenversorgung bereit stellen.

Zu diesem Zweck weisen die Funktionsmodule 11 komplementär zu den Führungsschienen 106 ausgebildete Einsteckvorsprünge 112 auf, so dass sie in die Führungsschienen 106 des Basismoduls 10 einführbar sind.

Insbesondere aus der Darstellung gemäß Figur 6 und 7 erkennt man, dass das Basismodul 10 an einem Endbereich der Führungsschienen 106 mit einem über die Rückseite 101 vorstehenden Anschlussbalken 107 ausgerüstet ist, der entsprechend des realisierten Rastermaßes eine Vielzahl von Schnittschnellen 107a trägt, zu denen die dem Anschlussbalken 107 zugewandte Stirnseite der Funktionsmodule 11 komplementär ausgebildet ist. Der Anschlussbalken 107 bildet außerdem einen Endanschlag für die in die Führungsschienen 106 einschiebbaren Funktionsmodule 11.

Insoweit ist es möglich, die gewünschte Art und Anzahl der Funktionsmodule 11 in die Führungsschienen 106 des Basismoduls einzuschieben, bis die Funktionsmodule 11 am Anschlussbalken 107 zur Anlage kommen, dort selbsttätig die Konnektierung mit den Schnittstellen 107a vornehmen und nachfolgend von der in der Basiseinheit 103 angeordneten Stromversorgungseinheit mit Betriebsstrom versorgt werden, woraufhin sich die Funktionsmodule 11 selbsttätig an der Datenverarbeitungseinrichtung in der Basiseinheit 103 anmelden und nachfolgend funktionsbereit sind und über die Anzeige- und Bedieneinheit 102 des Basismoduls steuerbar sind.

Beispielsweise können die Funktionsmodule 11 im dargestellten Ausführungsbeispiel ein Beatmungsmodul 11.2 für die Beatmung eines Patienten sowie einen Defibrillator 11.5 umfassen.

Ferner können auch weitere, korrespondierend zu den Funktionsmodulen ausgebildete Module, wie ein Akkumulator 11.1 für eine mobile Stromversorgung ohne Nutzung der Netzanschlussbuchsen 111, ein Drucker 11.3 für die Ausgabe von Messergebnissen, ein Patientenüberwachungsmodul 11.4 für die Überwachung der Vitalparameter des Patienten vorgesehen und gleichermaßen wie die Funktionsmodule an dem Basismodul befestigt und konnektiert werden.

Somit wird bei der in den Zeichnungen dargestellten Maximalkonfiguration ein kompaktes etwa quaderförmiges modulares Intensivtherapiegerät aus Basismodul 10 und den genannten Funktionsmodulen und korrespondierenden Modulen 11.1, 11.2, 11.3, 11.4, 11.5 ausgebildet, welches mittels des Handgriffes 108 leicht von einer Person tragbar ist.

Zur Sicherstellung der gewünschten kompakten quaderförmigen Form ragt überdies die fest mit dem Basismodul 10 verbundene Basiseinheit 103 in einer solchen Länge L über die Rückseite 101 des Basismoduls 10 hervor, welche der Breite B der einzelnen Gehäuse der Module 11.1 bis 11.5 entspricht.

Ein solchermaßen aus Funktionsmodulen, dazu korrespondierenden weiteren Modulen und einem Basismodul zusammengestelltes modulares Intensivtherapiegerät 1 kann nicht nur im Rahmen der Notfallversorgung am Patienten und im Rettungsfahrzeug eingesetzt werden, sondern nach Anlieferung eines Patienten an ein Krankenhaus auch zur stationären Weiterbehandlung des Patienten dienen (z. B. Notaufnahme, Intensivstation, Chirurgie), wozu es lediglich erforderlich ist, die Netzanschlussbuchse 111 für das 220 V Wechselstromnetz anzuschließen und eventuell das Beatmungsmodul 11.2 von einer Gasversorgung aus bereitgestellten Flaschen auf eine Druckleitung, z.B. die Sauerstoffversorgung des Krankenhauses, umzustellen. Das Beatmungsmodul 11.2 kann zu diesem Zweck turbinengetrieben sein.

Die im Rahmen der stationären Versorgung des Patienten dann nicht notwendige mobile Stromversorgung durch einen Akkumulator 11.1 kann beispielsweise gegen ein Funktionsmodul zur Verabreichung von Medikamenten oder dergleichen ausgetauscht werden, oder aber entfernt und wieder aufgeladen werden.

Wesentlich ist aber, dass der Patient nach seinem Eintreffen im Krankenhaus nicht neu an Intensivtherapiegeräte angeschlossen werden muss, sondern die im Rahmen der Notfallversorgung gelegten Anschlüsse und Verbindungen zu den bereits verwendeten Intensivtherapiegeräten weiter genutzt werden können.

Es versteht sich, dass anstelle der aus der Zeichnung dargestellten Ausgestaltung auch viele weitere Varianten mit mehr oder weniger Funktionsmodulen oder anderem Funktionsumfang im Rahmen der Erfindung denkbar sind.

## Patentansprüche

1. Modulares Intensivtherapiegerät (1), umfassend ein Basismodul (10) mit einer Vorderseite (100) und einer Rückseite (101) sowie mehrere an dem Basismodul (10) lösbar befestigbare und mit diesem elektrisch verbindbare Funktionsmodule (11), die eine intensivmedizinische Therapiefunktionalität zur aktiven Therapie akuter Störungen der Vitalfunktionen aufweisen, wobei im Bereich der Vorderseite (100) des Basismoduls (10) eine Anzeige- und Bedieneinheit (102) und im Bereich der Rückseite (101) des Basismoduls (10) Aufnahmevorrichtungen für die Funktionsmodule (11) angeordnet sind, wobei die Funktionsmodule (11) jeweils in separaten Gehäusen aufgenommen sind und das Basismodul (10) eine über die Rückseite (101) überstehende Basiseinheit (103) aufweist, die eine Stromversorgungseinheit und eine Datenverarbeitungseinheit umfasst, die mit der Anzeige- und Bedieneinheit (102) sowie mit den am Basismodul (10) befestigten und elektrisch verbundenen Funktionsmodulen (11) kommuniziert, wobei die Funktionsmodule (11) jeweils selbsttätig an der Datenverarbeitungseinheit anmeldbar sind und eine Kommunikation der angemeldeten Funktionsmodule (11) untereinander ermöglicht ist, so dass in Abhängigkeit von gemessenen Parametern eines Funktionsmodules (11) ein anderes Funktionsmodul (11) automatisch einstellbar ist und sämtliche Funktionsmodule (11) über die Anzeige- und Bedieneinheit (102) des Basismoduls (10) steuerbar sind und das Intensivtherapiegerät (1) als kompakte, von Hand tragbare Einheit ausgebildet ist, wobei die Funktionsmodule (11) ein Beatmungsmodul umfassen und das Beatmungsmodul eine Steuerung mit einer Schnittstelle aufweist, an die ein Narkosegerät anschließbar und mittels der Steuerung des Beatmungsmoduls steuerbar ist.

2. Modulares Intensivtherapiegerät (1) nach Anspruch 1, **dadurch gekennzeichnet, dass** die Basiseinheit (103) in einer der Breite (B) der Funktionsmodule (11) entsprechenden Länge (L) über die Rückseite (101) übersteht.

3. Modulares Intensivtherapiegerät (1) nach Anspruch 1 oder 2, **dadurch gekennzeichnet, dass** die Basiseinheit (103) die Unterseite des modularen Intensiv-therapiegerätes (1) bildet.

4. Modulares Intensivtherapiegerät (1) nach einem der Ansprüche 1 bis 3, **dadurch gekennzeichnet, dass** die Aufnahmevorrichtungen für die Funktionsmodule (11) eine Vielzahl von parallel zueinander im Bereich der Rückseite (101) des Basismoduls (10) verlaufende Führungsschienen (106) für komplementär ausgebildete Einsteckvorsprünge der Funktionsmodule (11) und einen mit elektrischen Verbindungsschnittstellen für die Funktionsmodule (11) ausgebildeten Anschlußbalken (107) umfassen, der einen Endanschlag für die in die Führungsschienen (106) einschiebbaren Funktionsmodule (11) bildet.

5. Modulares Intensivtherapiegerät (1) nach einem der Ansprüche 1 bis 4, **dadurch gekennzeichnet, dass** die Funktionsmodule (11) ein Defibrillatormodul, Infusionsmodul, Elektroenzephalografmodul und/oder ein Hochfrequenzchirurgiemodul umfassen.

6. Modulares Intensivtherapiegerät (1) nach einem der Ansprüche 1 bis 5, **dadurch gekennzeichnet, dass** es ein korrespondierend zu den Funktionsmodulen (11) ausgebildetes und alternativ oder zusätzlich zu den Funktionsmodulen (11) an einer Aufnahmevorrichtung des Basismoduls (10) befestigbares und mit diesem elektrisch verbindbares Patientenüberwachungsmodul zur Überwachung von Vitalfunktionen des Patienten und/oder ein Ultraschalldiagnostikmodul aufweist.

7. Modulares Intensivtherapiegerät (1) nach einem der Ansprüche 1 bis 6, **dadurch gekennzeichnet, dass** es einen korrespondierend zu den Funktionsmodulen (11) ausgebildeten und alternativ oder zusätzlich zu den Funktionsmodulen (11) an einer Aufnahmevorrichtung des Basismoduls (10) befestigbaren und mit diesem elektrisch verbindbaren Akkumulator für die mobile Stromversorgung des Intensivtherapiegerätes aufweist.

8. Modulares Intensivtherapiegerät (1) nach einem der Ansprüche 1 bis 7, **dadurch gekennzeichnet, dass** die Anzeige- und Bedieneinheit (102) einen Touchscreen umfasst.

## Claims

1. Modular intensive care device (1), comprising a base module (10) having a front side (100) and a rear side (101) and a plurality of functional modules (11) that are releasably fastenable to and electrically connectable to the base module (10), which functional modules (11) have an intensive care therapy functionality for the active treatment of acute disorders of the vital functions, wherein there are arranged in the region of the front side (100) of the base module (10) a display- and operating-unit and in the region of the rear side (101) of the base module (10) receiving devices for the functional modules (11), wherein the functional modules (11) are each accommodated in separate housings and the base module (10) comprises a base unit (103) that projects beyond the rear side (101), which base unit (103) comprises a power supply unit and a data processing unit which communicates with the display- and operating-unit (102) and with the functional modules (11) fastened to and electrically connected with the base module (10), wherein the functional modules (11) are each independently registrable at the data processing unit and communication of the registered functional modules (11) with each other is enabled, so that depending on measured parameters of one functional module (11) another functional module (11) is automatically engageable and all the functional modules (11) can be controlled by the display- and operating-unit (102) of the base module (10) and the intensive care unit (1) is formed as a compact, hand-portable unit, wherein the functional modules (11) comprise a ventilation module and the ventilation module comprises a controller with an interface to which an anaesthetic device can be connected and controlled by means of the controller of the ventilation module.

2. Modular intensive care device (1) according to claim 1, **characterised in that** the base unit (103) projects beyond the rear side (101) by a length (L) that corresponds to the width (B) of the functional modules (11).

3. Modular intensive care device (1) according to claim 1 or 2, **characterised in that** the base unit (103) forms the underside of the modular intensive care device (1).

4. Modular intensive care device (1) according to any one of claims 1 to 3, **characterised in that** the receiving devices for the functional modules (11) comprise a plurality of guide rails (106) for complementarily formed plug-in projections of the functional modules (11), which guide rails (106) extend parallel to each other in the region of the rear side (101) of the base module (10), and a connection bar (107) with electrical connection interfaces for the functional modules (11), which connection bar (107) forms an end stop for the functional modules (11) that can be inserted into the guide rails (106).

5. Modular intensive care device (1) according to any one of claims 1 to 4, **characterised in that** the functional modules (11) comprise a defibrillator module, infusion module, electroencephalograph module and/or a high frequency surgery module.

6. Modular intensive care device (1) according to any one of claims 1 to 5, **characterised in that** it comprises a patient monitoring module that corresponds in form to the functional modules (11) and is fastenable to and electrically connectable to a receiving device of the base module (10) instead of or in addition to the functional modules (11), for monitoring the vital functions of the patient, and/or an ultrasound diagnostic module.

7. Modular intensive care device (1) according to any one of claims 1 to 6, **characterised in that** it comprises a battery that corresponds in form to the functional modules (11) and is fastenable to and electrically connectable to a receiving apparatus of the base module (10) instead of or in addition to the functional modules (11), for the mobile supply of power to the intensive care device.

8. Modular intensive care device (1) according to any one of claims 1 to 7, **characterised in that** the display- and operating-unit (102) comprises a touch screen.

## Revendications

1. Appareil modulaire de soins intensifs (1), comprenant un module de base (10) avec une face avant (100) et une face arrière (101) ainsi que plusieurs modules fonctionnels (11) qui peuvent être fixés de manière amovible au module de base (10) et être reliés électriquement à celui-ci et qui présentent une fonctionnalité médicale de soins intensifs pour la thérapie active de perturbations aiguës des fonctions vitales,
dans lequel une unité d'affichage et de commande (102) est agencée dans la zone de la face avant (100) du module de base (10) et des dispositifs de logement destinés aux modules fonctionnels (11) sont agencés dans la zone de la face arrière (101) du module de base (10),
dans lequel les modules fonctionnels (11) sont logés à chaque fois dans des boîtiers séparés et le module de base (10) comporte une unité de base (103) qui dépasse de la face arrière (101) et qui comprend une unité d'alimentation en courant et une unité de traitement de données qui communique avec l'unité d'affichage et de commande (102) ainsi qu'avec les modules fonctionnels (11) fixés au module de base (10) et reliés électriquement à celui-ci,
dans lequel les modules fonctionnels (11) peuvent s'enregistrer à chaque fois automatiquement auprès de l'unité de traitement de données et une communication entre des modules fonctionnels enregistrés (11) est possible de telle sorte que, en fonction de paramètres mesurés d'un module fonctionnel (11), un autre module fonctionnel (11) peut être réglé automatiquement et tous les modules fonctionnels (11) peuvent être commandés par l'intermédiaire de l'unité d'affichage et de commande (102) du module de base (10) et l'appareil de soins intensifs (1) est conçu comme une unité compacte, portable à la main,
dans lequel les modules fonctionnels (11) comprennent un module respiratoire et le module respiratoire comporte une commande avec une interface à laquelle un appareil d'anesthésie peut être raccordé et être commandé au moyen de la commande du module respiratoire.

2. Appareil modulaire de soins intensifs (1) selon la revendication 1, **caractérisé en ce que** l'unité de base (103) dépasse de la face arrière (101) avec une longueur (L) correspondant à la largeur (B) des modules fonctionnels (11).

3. Appareil modulaire de soins intensifs (1) selon la revendication 1 ou 2, **caractérisé en ce que** l'unité de base (103) forme le dessous de l'appareil modulaire de soins intensifs (1).

4. Appareil modulaire de soins intensifs (1) selon l'une quelconque des revendications 1 à 3, **caractérisé en ce que** les dispositifs de logement destinés aux modules fonctionnels (11) comprennent une multiplicité de rails de guidage (106), qui s'étendent parallèlement les uns aux autres dans la zone de la face arrière (101) du module de base (10) et qui sont destinés à des parties en saillie d'enfichage, réalisées de manière complémentaire, des modules fonctionnels (11), et une barre de raccordement (107), qui est réalisée avec des interfaces de liaison électrique pour les modules fonctionnels (11) et qui forme une butée terminale pour les modules fonctionnels (11) pouvant être introduits dans les rails de guidage (106).

5. Appareil modulaire de soins intensifs (1) selon l'une quelconque des revendications 1 à 4, **caractérisé en ce que** les modules fonctionnels (11) comprennent un module défibrillateur, un module de perfusion, un module d'électroencéphalographie et/ou un module chirurgical à haute fréquence.

6. Appareil modulaire de soins intensifs (1) selon l'une quelconque des revendications 1 à 5, **caractérisé en ce qu'**il comporte un module de surveillance de patient réalisé de manière correspondante aux modules fonctionnels (11), pouvant être fixé à la place ou en plus des modules fonctionnels (11) à un dispositif de logement du module de base (10), pouvant être relié électriquement à celui-ci et destiné à la surveillance de fonctions vitales du patient et/ou un module d'échographie.

7. Appareil modulaire de soins intensifs (1) selon l'une quelconque des revendications 1 à 6, **caractérisé en ce qu'**il comporte un accumulateur réalisé de manière correspondante aux modules fonctionnels (11), pouvant être fixé à la place ou en plus des modules fonctionnels (11) à un dispositif de logement du module de base (10), pouvant être relié électriquement à celui-ci et destiné à l'alimentation en courant mobile de l'appareil de soins intensifs.

8. Appareil modulaire de soins intensifs (1) selon l'une quelconque des revendications 1 à 7, **caractérisé en ce que** l'unité d'affichage et de commande (102) comprend un écran tactile.
